# EUROPEAN PATENT SPECIFICATION

(11) **EP 3 197 521 B1**
(45) Date of publication and mention of the grant of the patent: **27.01.2021**
(21) Application number: 15843448.0
(22) Date of filing: 22.09.2015
(51) Int. Cl.: A61M 3/02

(54) **ANGULAR CAP FOR DISPENSING LIQUIDS**
ECKIGE KAPPE ZUR AUSGABE VON FLÜSSIGKEITEN
BOUCHON ANGULAIRE POUR DISTRIBUER DES LIQUIDES

(30) Priority: 22.09.2014 US 201462053515 P
(43) Date of publication of application: 02.08.2017
(73) Proprietor: Mehta, Ketan C., Santa Rosa, California 95403 (US)
(72) Inventor: Mehta, Ketan C., Santa Rosa, California 95403 (US)
(74) Representative: Boult Wade Tennant LLP
(86) International application number: PCT/US2015/051550
(87) International publication number: WO 2016/049082

(56) References cited:
- WO-A1-2013/124491
- WO-A1-2013/164759
- US-A- 2 135 052
- US-A- 2 330 019
- US-A- 2 330 019
- US-A- 2 608 841
- US-A- 5 788 683
- US-A- 6 156 017
- US-A1- 2009 247 941
- US-A1- 2009 247 941
- US-A1- 2010 282 246
- US-B1- 6 241 705
- US-B1- 6 540 718
- US-B1- 6 540 718

## Description

### CROSS REFERENCE TO RELATED APPLICATIONS

This application relates to, and claims the benefit of the earlier filing date and priority of U.S. Provisional Patent Application No. 62/053,515, filed on September 22, 2014, entitled "Angular Cap For Dispensing Liquids."

### FIELD OF THE INVENTION

The present invention relates generally to devices for dispensing liquids, including but not limited to lavage devices.

### BACKGROUND OF THE INVENTION

Liquid dispensing container/cap assemblies for dispensing liquids are known. For example, nasal rinsing and/or other body cavity irrigation assemblies and methods are disclosed in U.S. Patent Nos. 6,669,059 and 6,520,384. In these assemblies the cap may have a cylindrical lower portion, a rounded convex upper portion curving away from an axially aligned opening located in the uppermost surface of the upper portion, an open lower end, and a tubular conduit connected to the uppermost interior surface of the upper portion and having a hollow center axially aligned with the opening located in the upper portion. The container of these assemblies may have flexible sidewalls and an axially aligned neck configured to connect to the cap with a liquid tight connection. The conduit of the cap can extend into the container when the cap and container are joined together, or a flexible tube can be connected to the conduit, which flexible tube extends into the container.

The assemblies disclosed in the '059 and '384 patents may be used in the following manner. The user bends forward to a comfortable level, tilting the head slightly down and applies the cap snugly against the left nostril with the cap upper opening directed into the left nasal passage. The container may be squeezed to force liquid to enter the left nasal passage. The process is repeated applying the cap snugly against the right nostril. The liquid that was injected into the nasal passages will drain from the nasal passages or the mouth. The user then gently blows the nose. Any unused portion of the liquid may be discarded and the dispenser assembly should be cleaned. Thus, the assemblies disclosed in the '059 and '384 patents require the user or an assistant to squeeze the container to force liquid up into the nasal cavity as opposed to using gravity to drain the liquid out of the container.

The assemblies disclosed in the '059 and '384 patents are not effective for gravity feed of liquid to a user's nasal or other body cavity for at least two primary reasons. First, the conduit through which liquid flows out of the cap in these known assemblies is co-axial with the longitudinal axis of the liquid container. As a result, the degree to which the user would comfortably tilt her head to present her sinuses in the desired orientation to receive liquid for nasal rinsing, for example, would not permit the container to be optimally oriented to dispense liquid under the force of gravity. Accordingly, there is a need for a cap which permits the user's head to be tilted to the desired degree for nasal irrigation which also results in the liquid container being positioned in a preferred, substantially vertical, orientation for liquid dispensing under the force of gravity.

Second, the assemblies disclosed in the '059 and '384 patents do not include a cap which vents air into a container in a manner that would permit effective gravity feed of liquid to a user's nasal cavity from the container. In order for liquid to flow out of a fixed volume space (i.e., a container which is not squeezed) under the force of gravity, air must flow into the space to displace the liquid. The assemblies disclosed in the '059 and '384 patents do not permit such venting since there is only one opening in the cap through which liquid must flow out, and air must flow in. Indeed, the ability to vent air into a liquid container in a controlled, or even uncontrolled, manner when dispensing liquid under the influence of gravity is advantageous for all liquid dispensing activities, not just those associated with lavaging. Accordingly, there is a need for a cap which permits liquid to flow out and air to flow in to a liquid container simultaneously to allow effective dispensing of a liquid using gravity.

For the foregoing reasons, there is a need for apparatus and methods for dispensing a liquid to a nasal cavity in particular, but not limited to such use, which is simple to use, effective, and relatively inexpensive.

### Summary of the Invention

US 6,540,718 describes an appliance for the rinsing of nasal cavities.

US 2009/247941 describes a nasal irrigator that includes a connection mechanism, an air vent and a conduit.

Responsive to the foregoing challenges, Applicant has developed an innovative cap as described in claim 1.

It is to be understood that both the foregoing general description and the following detailed description are exemplary and explanatory only, and are not restrictive of the invention as claimed.

### Brief Description of the Drawings

In order to assist the understanding of this invention, reference will now be made to the appended drawings, in which like reference characters refer to like elements. The drawings are exemplary only, and should not be construed as limiting the invention.
Figure 1 is a pictorial view of a container and cap assembly in accordance with an embodiment of the present invention.
Figure 2 is a cross-sectional view of a container and cap assembly in accordance with an embodiment of the present invention.
Figure 3A is a rear elevation view of a container and cap assembly in accordance with an embodiment of the present invention.
Figure 38 is a front elevation view of a container and cap assembly in accordance with an embodiment of the present invention.

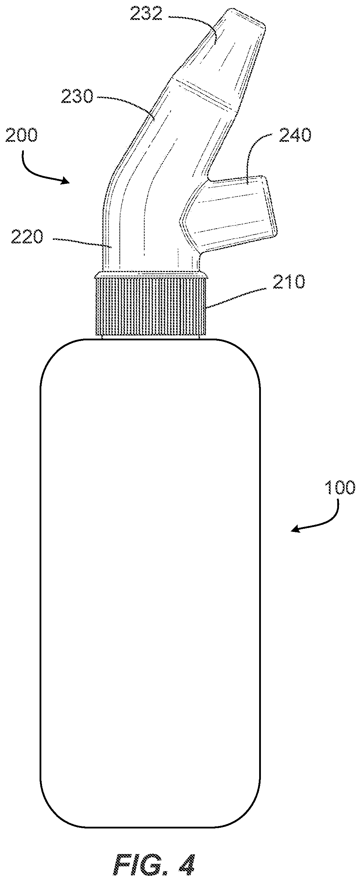

Figure 4 is a side elevation view of a container and cap assembly in accordance with an embodiment of the present invention.

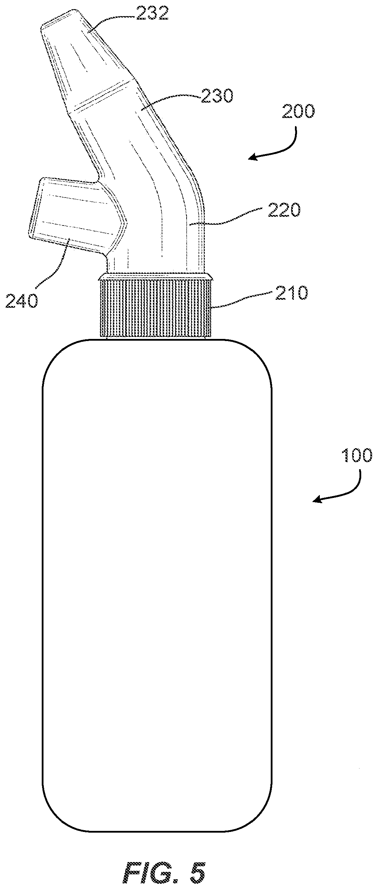

Figure 5 is an opposing side elevation view of a container and cap assembly in accordance with an embodiment of the present invention.

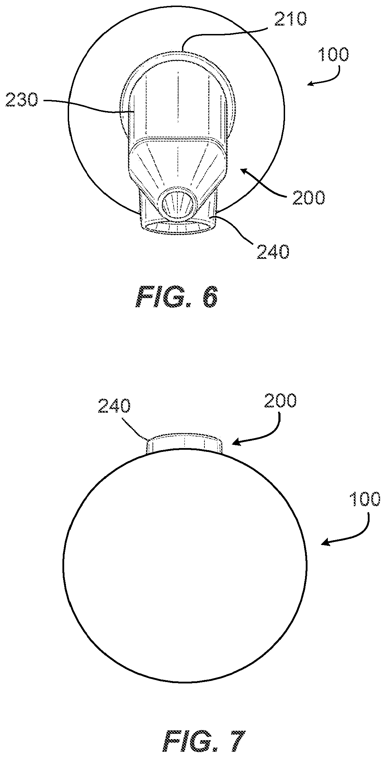

Figure 6 is a top plan view of a container and cap assembly in accordance with an embodiment of the present invention.
Figure 7 is a bottom plan view of a container and cap assembly in accordance with an embodiment of the present invention.

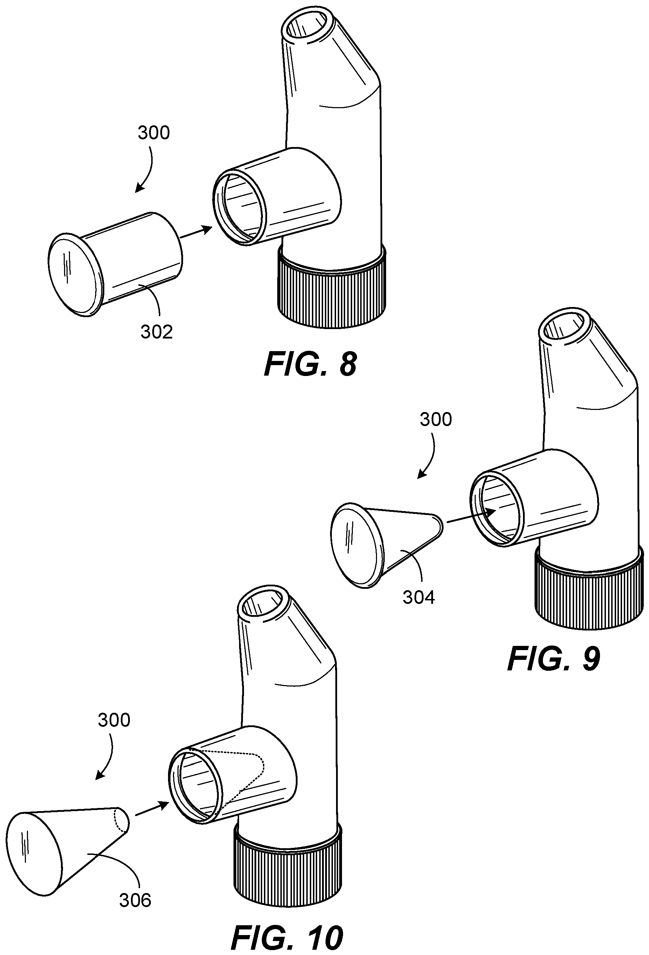

Figure 8 is a pictorial view of a container and cap assembly including an obturator/stopper in accordance with a first alternative embodiment of the present invention.
Figure 9 is a pictorial view of a container and cap assembly including an obturator/stopper in accordance with a second alternative embodiment of the present invention.
Figure 10 is a pictorial view of a container and cap assembly including an obturator/stopper in accordance with a third alternative embodiment of the present invention.

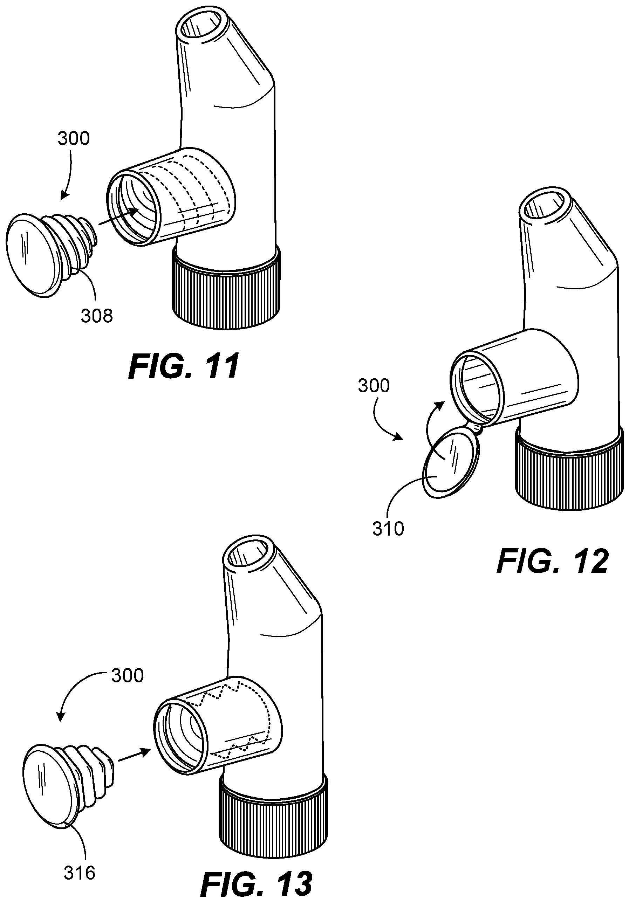

Figure 11 is a pictorial view of a container and cap assembly including an obturator/stopper in accordance with a fourth alternative embodiment of the present invention.
Figure 12 is a pictorial view of a container and cap assembly including an obturator/stopper in accordance with a fifth alternative embodiment of the present invention.
Figure 13 is a pictorial view of a container and cap assembly including an obturator/stopper in accordance with a sixth alternative embodiment of the present invention.

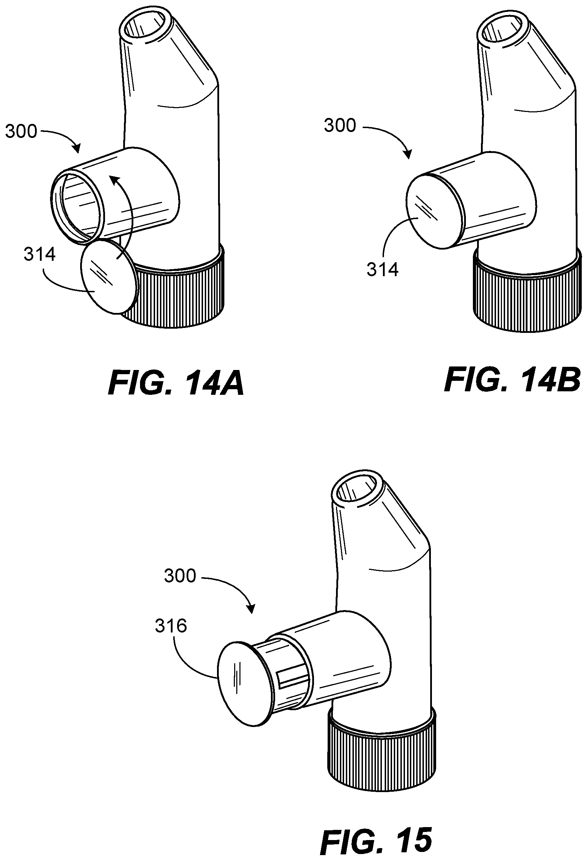

Figures 14A-14B are pictorial views of a container and cap assembly including an obturator/stopper in accordance with a seventh alternative embodiment of the present invention.
Figure 15 is a pictorial view of a container and cap assembly including an obturator/stopper in accordance with an eighth alternative embodiment of the present invention.

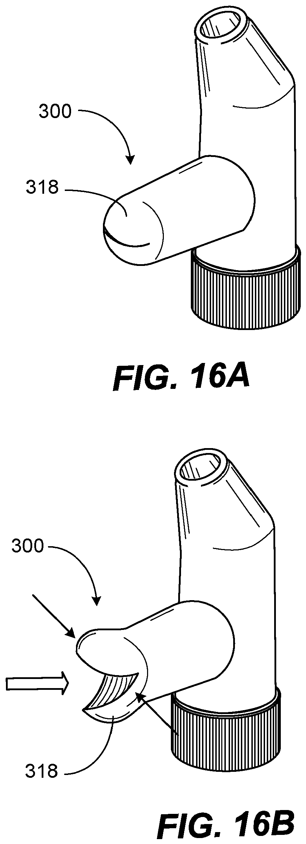

Figures 16A-16B are pictorial views of a container and cap assembly including an obturator/stopper in accordance with a ninth alternative embodiment of the present invention.

### DETAILED DESCRIPTION OF EMBODIMENTS OF THE INVENTION

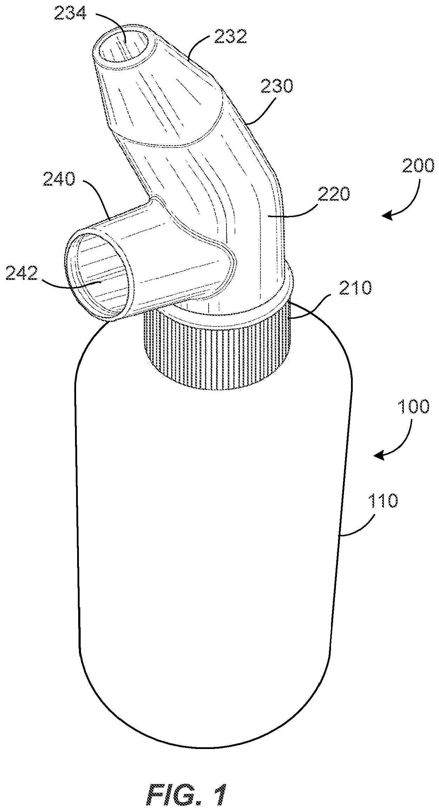

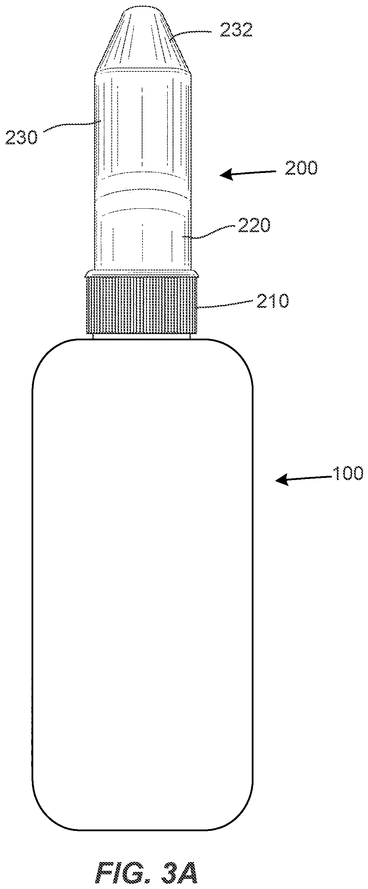

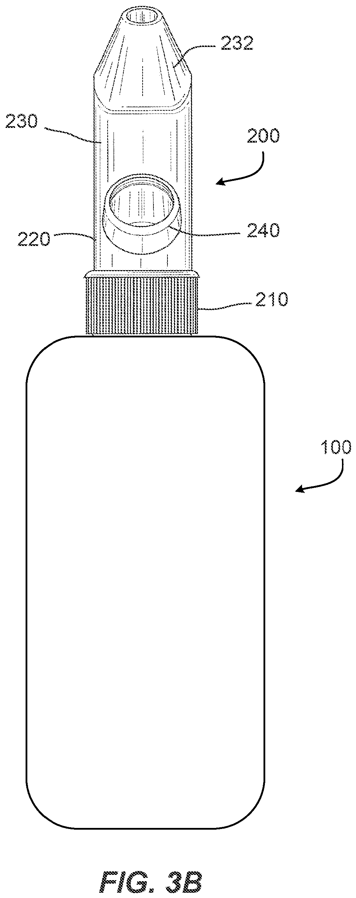

Reference will now be made in detail to apparatus embodiments of the present invention, examples of which are illustrated in the accompanying drawings. With reference to Figs. 1-7, in a first embodiment of the invention, the throat wall 130 of a container 100 is connected to the base portion 210 of the elongated body of a cap 200. The throat wall 130 may be connected to the base portion 210 by any of a number of means, including but not limited to screw threads or snap-on features. The container 100 may include an outer wall 110 which defines a space 120 for containing liquid. The liquid may be dispensed from the space 120 to the cap 200 via the container throat 132. The container 100 is preferably sized to be hand-held.

The cap 200 includes an elongated body 202 defining a first channel 222 extending from an opening in the base portion 210, through a neck portion 220, and to an opening in a tip portion 230 of the elongated body. The cap 200 further includes a side body 240 defining a second channel 242 extending from the first channel 222 near the neck portion 220 of the elongated body to an opening at a distal end of the side body. The tip portion 230 includes a terminus 232 which is sloped inward so as to have a reduced dimension at its most distal end. The shape of the terminus may be adapted to be received comfortably in a human body opening, such as, for example, a nostril or ear. The foregoing reference to "near the neck portion" is intended to include the portions of the cap 200 between the base 210 and the tip portion terminus 232.

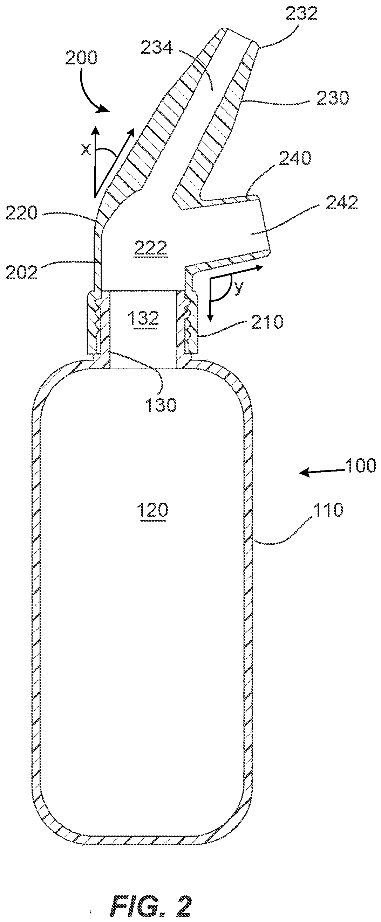

With particular reference to Fig. 2, the tip portion 230 has an axial dimension that extends at an angle x relative to an axial dimension of the neck portion 220, wherein angle x is in the range of between zero (0) and ninety (90) degrees. The side body 240 may have an axial dimension that extends at an obtuse angle ***y*** relative to an axial dimension of the neck portion 220. The angle ***x*** may be selected to permit the tip portion 230 to be comfortably and snuggly received in a user's body cavity, such as a nostril or ear, while at the same time permitting the container 100 to be oriented such that liquid will flow from the container under the influence of gravity. The angle ***y*** may be selected to permit a user to control the flow of air into the second channel 242 by hand when the container 100 is oriented such that liquid will flow from the container under the influence of gravity. Such control may be exercised by selectively covering the distal opening of the channel 242 with a finger, or with an obturator or stopper (explained below). It is appreciated that one or both of the tip portion 230 and the side body 240 may be flexible and capable of being adjusted to form various different angles x and y relative to the axial direction of the neck portion 220.

The relative diameters of the first channel 222 in the neck portion, the first channel in the tip portion 230, and the second channel 242, may be sized/adapted to permit liquid to flow from the base portion 210 of the cap 200 through the tip portion 230 under the influence of gravity while at the same time permitting air to flow into the first channel from the second channel. For example, to this end, the first channel 222 may have a reduced diameter 234 within the tip portion 230 relative to the diameter of the first channel in the neck portion 220. In a further example, the diameter of the second channel 242 may be less than the diameter of the first channel 222. In a still further example, the reduced diameter 234 within the tip portion 230 may be less than the diameter of the second channel 242. In a still further example, the diameter of the second channel 242 may vary over its length, as shown in Fig. 10.

With reference to Figs. 8-16B, the cap may also include an obturator or stopper 300 which may be provided by any of a number of different shapes, sizes and designs. Each of the illustrated obturators or stoppers may be shaped and sized to cooperate with the shape of the second channel 242 and side body 240 so as to selectively block the second channel in whole or in part. Preferably, the obturator or stopper may be adapted to be manipulated by hand to control the flow of air into the second channel 242. For example, the obturator or stopper may have a plug shape (Fig. 8, 302), a rounded cone shape (Fig. 9, 304), a frusto-conical shape (Fig. 10, 306), a threaded conical shape (Fig. 11, 308), a undulated shape (Fig. 13, 312), or a forked plug shape (Fig.15, 316). In each of the foregoing examples, the obturator or stopper 300 may be detachable from the side body 240 of the cap 200. In further examples, the obturator or stopper 300 may be permanently connected to the side body 240 of the cap 200 by a hinge (Fig. 12, 310) and (Figs. 14A-14B, 314). In a still further example, the obturator or stopper 300 may comprise an open and closable mouth (Figs. 16A-16B, 318) which is permanently connected to the side body 240.

The afore-described cap 200 may improve upon or even maximize the flow of a solution or liquid out of the container 100. The following is an example of how the cap 200 may be used for a tissue irrigation application. An irrigation solution may be prepared and provided in the container 100. The cap 200 may be connected to the container 100. The terminus 232 of the tip portion 230 may be inserted into a nasal passage, for example, on the right side (for the purposes of this example, the opening is initially inserted into the right side, but the user may begin the treatment in either side, as applicable). Next the user may tilt the head along with inserted cap to the left side such that the container 100 is inverted all while the second channel 242 of the cap 200 is blocked by a finger, obturator, or stopper. The finger, obturator, or stopper may be fully or partially removed from the second channel 242 at this point to allow solution to enter the nasal passage on the right side and come out from the left side. The user need not squeeze the container 100 since the solution should flow under the influence of gravity. The user can continue irrigation as long as needed, and the same procedure can be applied for the other nasal passage. Manipulation of the user's finger, or of the obturator or stopper may be used to regulate the flow rate of the solution out of the container 100 by regulating the flow of air into the second channel 242. If the user requires additional force of irrigation, the user can squeeze the container 100 and use a finger, obturator or plug to block solution from coming out of the second channel 242. The diameter of the second channel 242 may be selected and controlled, but not necessarily, so that the container 100 may empty under the influence of gravity in a matter of seconds. The cap 200 may be designed to facilitate the smooth flow of liquids having various viscosities, weights and temperatures. The cap 200 may be manufactured as a single component, or it may be comprised of multiple components assembled as needed.

The functional elements and method steps described above are provided as illustrative examples of one technique for implementing the invention; one skilled in the art will recognize that many other implementations are possible without departing from the present invention as recited in the claims. Accordingly, the disclosure of the present invention is intended to be illustrative, but not limiting, of the scope of the invention. It is intended that the present invention cover all such modifications and variations of the invention, provided they come within the scope of the appended claims.

## Claims

1. A cap (200) comprising:
an elongated body (202) defining a first channel (222) extending from a first opening in a base portion (210) of the elongated body (202) through a neck portion (220) of the elongated body to a second opening (234) in a tip portion (230) of the elongated body (202);
a side body (240) defining a second channel (242) extending from the first channel (222) near the neck portion of the elongated body to a third opening at a distal end of the side body (240),
wherein a terminus (232) of the tip portion (230) is sloped inward in a manner adapted to be received in a human body opening,
wherein an axial dimension of the tip portion extends at an angle relative to an axial dimension of the neck portion;
wherein the base portion (210) is adapted to couple to a hand-held container (100);
**characterised in that** the cap (200) defines:
a longitudinal axis through a center of the first opening in the base portion (210), and
a first plane through the first channel (222) and the second channel (242),
wherein the tip portion is angled with respect to the neck portion so that a first portion of an exterior surface of the elongated body (202) in the first plane forms a first acute angle with the longitudinal axis, and a second portion of the exterior surface of the elongated body (202), diametrically opposed to the first portion of the exterior surface of the elongated body (202), forms a second acute angle with an exterior surface of the side body in the first plane.

2. The cap (200) of claim 1, wherein the first channel (222) has a reduced diameter within the tip portion (230) relative to a diameter in the neck portion (220).

3. The cap (200) of claim 2, wherein a diameter of the second channel (242) is less than a diameter of the first channel (222).

4. The cap (200) of claim 2, wherein relative diameters of the first channel (222) and the second channel (242) are adapted to permit liquid to flow from the base portion (210) to the tip portion (230) under the influence of gravity while at the same time permitting air to flow into the first channel (222) from the second channel (242).

5. The cap (200) of claim 4, further comprising an obturator or stopper (300), and wherein the second channel (242) is shaped to be selectively blocked in whole or in part by the obturator or stopper (300).

6. The cap (200) of claim 5, wherein the obturator or stopper (300) is adapted to be manipulated by hand to control the flow of air into the second channel (242).

7. The cap (200) of claim 5, wherein the obturator or stopper (300) is permanently connected to the side body (240).

8. The cap (200) of claim 5, wherein the second channel (242) is shaped to fixedly receive the obturator or stopper (300).

9. The cap (200) of claim 4, wherein an axial dimension of the side body (240) extends at an obtuse angle relative to the longitudinal axis.

10. The cap (200) of claim 1, wherein a diameter of the second channel (242) is less than a diameter of the first channel (222).

11. The cap (200) of claim 1, wherein relative diameters of the first channel (222) and the second channel (242) are adapted to permit liquid to flow from the base portion (210) to the tip portion (230) under the influence of gravity while at the same time permitting air to flow into the first channel (222) from the second channel (242).

12. The cap (200) of claim 1, further comprising an obturator or stopper (300), and wherein the second channel (242) is shaped to be selectively blocked in whole or in part by the obturator or stopper (300).

13. The cap (200) of claim 12, wherein the obturator or stopper (300) is adapted to be manipulated by hand to control the flow of air into the second channel (242).

14. The cap (200) of claim 12, wherein the obturator or stopper (300) is permanently connected to the side body (240).

15. The cap (200) of claim 12, wherein the second channel (242) is shaped to fixedly receive the obturator or stopper (300).

16. The cap (200) of claim 1, wherein an axial dimension of the side body (240) extends at an obtuse angle relative to the longitudinal axis.

17. The cap (200) of claim 16, further comprising an obturator or stopper (300), and wherein the second channel (242) is shaped to be selectively blocked in whole or in part by the obturator or stopper (300).

18. The cap (200) of claim 17, wherein the obturator or stopper (300) is adapted to be manipulated by hand to control the flow of air into the second channel (242).

19. The cap (200) of claim 17, wherein the obturator or stopper (300) is permanently connected to the side body (240).

20. The cap (200) of claim 17, wherein the second channel (242) is shaped to fixedly receive the obturator or stopper (300).

21. The cap (200) of claim 1, wherein the cap (200) defines a second plane orthogonal to the first plane, the first plane and second plane including the longitudinal axis, and the second opening and the third opening are on a common side of the second plane.

## Patentansprüche

1. Kappe (200), umfassend:
einen länglichen Körper (202), welcher einen ersten Kanal (222) definiert, welcher sich von einer ersten Öffnung in einem Basis-Abschnitt (210) des länglichen Körpers (202) durch einen Nacken-Abschnitt (220) des länglichen Körpers zu einer zweiten Öffnung (234) in einem Spitzen-Abschnitt (230) des länglichen Körpers (202) erstreckt;
einen Seiten-Körper (240), welcher einen zweiten Kanal (242) definiert, welcher sich von dem ersten Kanal (222) nahe dem Nacken-Abschnitt des länglichen Körpers zu einer dritten Öffnung an einem distalen Ende des Seiten-Körpers (240) erstreckt,
wobei ein Endpunkt (232) des Spitzen-Abschnitts (230) in einer Weise nach innen geneigt ist, dass er dazu eingerichtet ist, in einer menschlichen Körperöffnung aufgenommen zu werden,
wobei sich eine axiale Abmessung des Spitzen-Abschnitts bei einem Winkel relativ zu einer axialen Abmessung des Nacken-Abschnitts erstreckt;
wobei der Basis-Abschnitt (210) dazu eingerichtet ist, mit einem handgehaltenen Behälter (100) zu koppeln;
**dadurch gekennzeichnet, dass** die Kappe (200) definiert:
eine longitudinale Achse durch ein Zentrum der ersten Öffnung in dem Basis-Abschnitt (210), und
eine erste Ebene durch den ersten Kanal (222) und den zweiten Kanal (242),
wobei der Spitzen-Abschnitt in Bezug auf den Nacken-Abschnitt gewinkelt ist, so dass ein erster Abschnitt einer äußeren Fläche des länglichen Körpers (202) in der ersten Ebene einen ersten spitzen Winkel mit der longitudinalen Achse bildet, und dass ein zweiter Abschnitt der äußeren Fläche des länglichen Körpers (202), welcher dem ersten Abschnitt der äußeren Fläche des länglichen Körpers (202) diametral entgegengesetzt ist, einen zweiten spitzen Winkel mit einer äußeren Fläche des Seiten-Körpers in der ersten Ebene bildet.

2. Kappe (200) nach Anspruch 1, wobei der erste Kanal (222) einen reduzierten Durchmesser innerhalb des Spitzen-Abschnitts (230) relativ zu einem Durchmesser in dem Nacken-Abschnitt (220) aufweist.

3. Kappe (200) nach Anspruch 2, wobei ein Durchmesser des zweiten Kanals (242) geringer ist als ein Durchmesser des ersten Kanals (222).

4. Kappe (200) nach Anspruch 2, wobei relative Durchmesser des ersten Kanals (222) und des zweiten Kanals (242) dazu eingerichtet sind, einer Flüssigkeit zu erlauben, von dem Basis-Abschnitt (210) zu dem Spitzen-Abschnitt (230) unter dem Einfluss der Schwerkraft zu strömen, während zu der gleichen Zeit Luft erlaubt wird, in den ersten Kanal (222) von dem zweiten Kanal (242) zu strömen.

5. Kappe (200) nach Anspruch 4, ferner umfassend ein Blockierungselement oder ein Stoppelement (300), und wobei der zweite Kanal (242) derart geformt ist, selektiv vollständig oder teilweise durch das Blockierungselement oder das Stoppelement (300) blockiert zu werden.

6. Kappe (200) nach Anspruch 5, wobei das Blockierungselement oder das Stoppelement (300) dazu eingerichtet ist, händisch manipuliert zu werden, um die Strömung von Luft in den zweiten Kanal (242) zu steuern.

7. Kappe (200) nach Anspruch 5, wobei das Blockierungselement oder das Stoppelement (300) mit dem Seiten-Körper (240) permanent verbunden ist.

8. Kappe (200) nach Anspruch 5, wobei der zweite Kanal (242) geformt ist, das Blockierungselement oder das Stoppelement (300) fest aufzunehmen.

9. Kappe (200) nach Anspruch 4, wobei sich eine axiale Abmessung des Seiten-Körpers (240) in einem stumpfen Winkel relativ zu der longitudinalen Achse erstreckt.

10. Kappe (200) nach Anspruch 1, wobei ein Durchmesser des zweiten Kanals (242) geringer ist als ein Durchmesser des ersten Kanals (222).

11. Kappe (200) nach Anspruch 1, wobei relative Durchmesser des ersten Kanals (222) und des zweiten Kanals (242) dazu eingerichtet sind, einer Flüssigkeit zu erlauben, von dem Basis-Abschnitt (210) zu dem Spitzen-Abschnitt (230) unter dem Einfluss der Schwerkraft zu strömen, während zu der gleichen Zeit Luft erlaubt wird, in den ersten Kanal (222) von dem zweiten Kanal (242) zu strömen.

12. Kappe (200) nach Anspruch 1, ferner umfassend ein Blockierungselement oder ein Stoppelement (300), und wobei der zweite Kanal (242) derart geformt ist, selektiv vollständig oder teilweise durch das Blockierungselement oder das Stoppelement (300) blockiert zu werden.

13. Kappe (200) nach Anspruch 12, wobei das Blockierungselement oder das Stoppelement (300) dazu eingerichtet ist, händisch manipuliert zu werden, um die Strömung von Luft in den zweiten Kanal (242) zu steuern.

14. Kappe (200) nach Anspruch 12, wobei das Blockierungselement oder das Stoppelement (300) mit dem Seiten-Körper (240) permanent verbunden ist.

15. Kappe (200) nach Anspruch 12, wobei der zweite Kanal (242) geformt ist, das Blockierungselement oder das Stoppelement (300) fest aufzunehmen.

16. Kappe (200) nach Anspruch 1, wobei sich eine axiale Abmessung des Seiten-Körpers (240) in einem stumpfen Winkel relativ zu der longitudinalen Achse erstreckt.

17. Kappe (200) nach Anspruch 16, ferner umfassend ein Blockierungselement oder ein Stoppelement (300), und wobei der zweite Kanal (242) derart geformt ist, selektiv vollständig oder teilweise durch das Blockierungselement oder das Stoppelement (300) blockiert zu werden.

18. Kappe (200) nach Anspruch 17, wobei das Blockierungselement oder das Stoppelement (300) dazu eingerichtet ist, händisch manipuliert zu werden, um die Strömung von Luft in den zweiten Kanal (242) zu steuern.

19. Kappe (200) nach Anspruch 17, wobei das Blockierungselement oder das Stoppelement (300) mit dem Seiten-Körper (240) permanent verbunden ist.

20. Kappe (200) nach Anspruch 17, wobei der zweite Kanal (242) geformt ist, das Blockierungselement oder das Stoppelement (300) fest aufzunehmen.

21. Kappe (200) nach Anspruch 1, wobei die Kappe (200) eine zweite Ebene, orthogonal zu der ersten Ebene, definiert, wobei die erste Ebene und die zweite Ebene die longitudinale Achse umfassen, und wobei die zweite Öffnung und die dritte Öffnung an einer gemeinsamen Seite der zweiten Ebene sind.

## Revendications

1. Capuchon (200) comprenant :
un corps allongé (202) définissant un premier canal (222) s'étendant à partir d'une première ouverture dans une partie de base (210) du corps allongé (202) à travers une partie de col (220) du corps allongé vers une deuxième ouverture (234) dans une partie de pointe (230) du corps allongé (202) ;
un corps latéral (240) définissant un second canal (242) s'étendant à partir du premier canal (222) près de la partie de col du corps allongé vers une troisième ouverture au niveau d'une extrémité distale du corps latéral (240),
dans lequel une terminaison (232) de la partie de pointe (230) est inclinée vers l'intérieur d'une manière adaptée pour être reçue dans une ouverture de corps humain,
dans lequel une dimension axiale de la partie de pointe s'étend sur un angle par rapport à une dimension axiale de la partie de col ;
dans lequel la partie de base (210) est adaptée pour se coupler à un contenant portatif (100) ;
**caractérisé en ce que** le capuchon (200) définit :
un axe longitudinal à travers un centre de la première ouverture dans la partie de base (210), et
un premier plan à travers le premier canal (222) et le second canal (242),
dans lequel la partie de pointe est en angle par rapport à la partie de col de sorte qu'une première partie d'une surface extérieure du corps allongé (202) dans le premier plan forme un premier angle aigu avec l'axe longitudinal, et une seconde partie de la surface extérieure du corps allongé (202), diamétralement opposée à la première partie de la surface extérieure du corps allongé (202), forme un second angle aigu avec une surface extérieure du corps latéral dans le premier plan.

2. Capuchon (200) selon la revendication 1, dans lequel le premier canal (222) a un diamètre réduit à l'intérieur de la partie de pointe (230) par rapport à un diamètre dans la partie de col (220).

3. Capuchon (200) selon la revendication 2, dans lequel un diamètre du second canal (242) est inférieur à un diamètre du premier canal (222).

4. Capuchon (200) selon la revendication 2, dans lequel des diamètres relatifs du premier canal (222) et du second canal (242) sont adaptés pour permettre à du liquide de s'écouler à partir de la partie de base (210) vers la partie de pointe (230) sous l'influence de la gravité tout en permettant en même temps à de l'air de s'écouler dans le premier canal (222) à partir du second canal (242).

5. Capuchon (200) selon la revendication 4, comprenant en outre un obturateur ou un bouchon (300), et dans lequel le second canal (242) est mis en forme pour être sélectivement bloqué totalement ou partiellement par l'obturateur ou le bouchon (300).

6. Capuchon (200) selon la revendication 5, dans lequel l'obturateur ou le bouchon (300) est adapté pour être manipulé à la main afin de commander l'écoulement de l'air dans le second canal (242).

7. Capuchon (200) selon la revendication 5, dans lequel l'obturateur ou le bouchon (300) est relié de manière permanente au corps latéral (240).

8. Capuchon (200) selon la revendication 5, dans lequel le second canal (242) est mis en forme pour recevoir de manière fixe l'obturateur ou le bouchon (300).

9. Capuchon (200) selon la revendication 4, dans lequel une dimension axiale du corps latéral (240) s'étend selon un angle obtus par rapport à l'axe longitudinal.

10. Capuchon (200) selon la revendication 1, dans lequel un diamètre du second canal (242) est inférieur à un diamètre du premier canal (222).

11. Capuchon (200) selon la revendication 1, dans lequel des diamètres relatifs du premier canal (222) et du second canal (242) sont adaptés pour permettre à du liquide de s'écouler à partir de la partie de base (210) vers la partie de pointe (230) sous l'influence de la gravité tout en permettant en même temps à de l'air de s'écouler dans le premier canal (222) à partir du second canal (242).

12. Capuchon (200) selon la revendication 1, comprenant en outre un obturateur ou un bouchon (300), et dans lequel le second canal (242) est mis en forme pour être sélectivement bloqué totalement ou partiellement par l'obturateur ou le bouchon (300).

13. Capuchon (200) selon la revendication 12, dans lequel l'obturateur ou le bouchon (300) est adapté pour être manipulé à la main pour commander l'écoulement d'air dans le second canal (242).

14. Capuchon (200) selon la revendication 12, dans lequel l'obturateur ou le bouchon (300) est relié de manière permanente au corps latéral (240).

15. Capuchon (200) selon la revendication 12, dans lequel le second canal (242) est mis en forme pour recevoir de manière fixe l'obturateur ou le bouchon (300).

16. Capuchon (200) selon la revendication 1, dans lequel une dimension axiale du corps latéral (240) s'étend selon un angle obtus par rapport à l'axe longitudinal.

17. Capuchon (200) selon la revendication 16, comprenant en outre un obturateur ou un bouchon (300), et dans lequel le second canal (242) est mis en forme pour être sélectivement bloqué totalement ou partiellement par l'obturateur ou le bouchon (300).

18. Capuchon (200) selon la revendication 17, dans lequel l'obturateur ou le bouchon (300) est adapté pour être manipulé à la main pour commander l'écoulement d'air dans le second canal (242).

19. Capuchon (200) selon la revendication 17, dans lequel l'obturateur ou le bouchon (300) est relié de manière permanente au corps latéral (240).

20. Capuchon (200) selon la revendication 17, dans lequel le second canal (242) est mis en forme pour recevoir de manière fixe l'obturateur ou le bouchon (300).

21. Capuchon (200) selon la revendication 1, dans lequel le capuchon (200) définit un second plan orthogonal au premier plan, le premier plan et le second plan comprenant l'axe longitudinal, et la deuxième ouverture et la troisième ouverture sont sur un côté commun du second plan.
